# EUROPEAN PATENT APPLICATION

(11) **EP 1 310 569 A2**
(43) Date of publication of application: **14.05.2003**
(21) Application number: 02445148.6
(22) Date of filing: 07.11.2002
(51) Int. Cl.: C12Q 1/68

(54) **Method and test kit for the detection of genes**

(30) Priority: 09.11.2001 JP 2001345133
(71) Applicant: President of Gifu University, Gifu-shi, Gifu 501-1193 (JP)
(72) Inventor: Ezaki, Takayuki, Gifu-shi, Gifu 500-8454 (JP)
(74) Representative: Lindberg, Olle Nils Olof

(57) **Abstract**

A test kit (100) that enables to rapidly specify a true pathogen among a plurality of pathogen candidates suspected of having caused infectious disease. The test kit includes a primer reagent (10) obtained by mixing 10 to 100 kinds of amplifying primers, and a DNA microarray (20) having spots (22) of DNAs having a base sequence to be amplified by each of the amplifying primers.

## Description

The present invention relates to a method and a test kit for detecting a polynucleotide (gene) having a target base sequence in a test sample.

According to the conventional Multiplex PCR (Polymerase Chain Reaction) method, a primer reagent obtained by mixing 2 to 4 kinds of PCR primers designed so as to amplify genes having a different length, and a test sample containing a gene functioning as a template for the PCR reaction are PCR-reacted. By subjecting the PCR product (gene) produced by the PCR reaction to electrophoresis using agarose gel and staining it, the PCR product is visualized. According to the Multiplex PCR method, the kind of an amplified gene, that is, a gene contained in the test sample is specified based on the length of the visualized PCR product.

However, as the kind of PCR primers contained in a primer reagent is increased, a noise on an electrophoresis gel is increased. For this reason, when a primer reagent containing a plurality of kinds of PCR primers is used, it is difficult to specify the amplified gene. In order to maintain sufficient detection sensitivity, inclusion of maximum of 4 kinds of PCR primers in a primer reagent is a limit. Therefore, it has not been reported to use 10 or more kinds of PCR primers.

Conventionally, it has been necessary to design the PCR product amplified by each PCR primer so as to be distinguishable on an electrophoresis gel having a limited developing width. However, it has been remarkably troublesome to suitably perform design of a PCR primer. For this reason, there has been limitation in the kind of PCR primers which can be PCR-reacted at once.

An object of the present invention is to provide a method of easily and rapidly determining whether a polynucleotide containing a target base sequence is present in a test sample.

To achieve the above object, the present invention provides a method for determining whether a polynucleotide having a target base sequence is present in a test sample. The method includes preparing a gene detecting primer reagent by mixing at least ten kinds of amplifying primers, preparing a DNA microarray by spotting DNAs having a base sequence to be amplified by the amplifying primers, respectively, or DNAs substantially identical to the DNAs on a substrate, extracting a sample extract from the test sample, amplifying a gene by performing the PCR reaction using the sample extract and the gene detecting primer reagent, and detecting the PCR reaction product using the DNA microarray.

A further perspective of the present invention is a gene detecting primer reagent for detecting a polynucleotide having a target base sequence in a test sample. The reagent is a mixture of at least ten kinds of amplifying primers.

A further perspective of the present invention is a DNA microarray for detecting a polynucleotide having a target base sequence in a test sample, The microarray includes a substrate, and spots of DNAs having a base sequence to be amplified by each of 10 or more kinds of amplifying primers, or of DNAs substantially identical to the DNAs, formed on the substrate.

A further perspective of the present invention is a test kit for detecting a polynucleotide having a target base sequence in a test sample. The test kit includes a primer reagent obtained by mixing at least ten kinds of amplifying primers and a DNA microarray having spots of DNAs having a base sequence to be amplified by each of the amplifying primers, or of DNAs substantially identical to the DNAs.

A further perspective of the present invention is a method of manufacturing a test kit for detecting a polynucleotide having a target base sequence in a test sample. The method includes preparing a gene detecting primer reagent by mixing aL least ten kinds of amplifying primers, and preparing a DNA microarray by spotting DNAs having a base sequence to be amplified by the amplifying primers, or DNAs substantially identical to the DNAs on a substrate.

Other aspects and advantages of the present invention will become apparent from the following description, taken in conjunction with the accompanying drawings, illustrating by way of example the principles of the invention.

The features of the present invention that are believed to be novel are set forth with particularity in the appended claims. The invention, together with objects and advantages thereof, may best be understood by reference to the following description of the presently preferred embodiments together with the accompanying drawings in which:

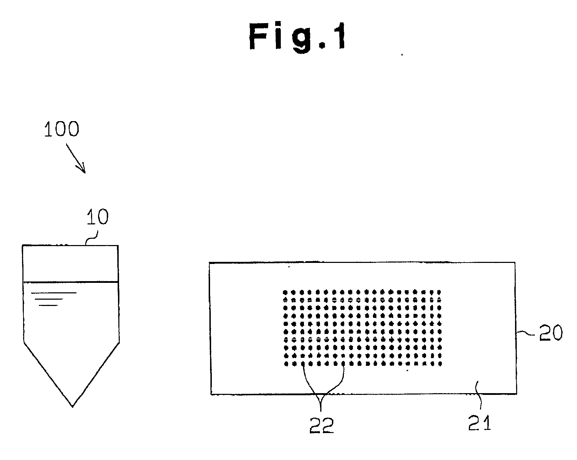

Fig. 1 is a schematic view of a test kit in accordance with one embodiment of the present invention.

A gene detecting method in accordance with one embodiment of the present invention will be explained below.

According to a gene detecting method of the present embodiment, whether a polynucleotide having a target base sequence is present in a test sample potentially containing a polynucleotide, is determined. According to the gene detecting method, extremely many kinds of polynucleotide (for example, a gene having a target base sequence) can be detected by one time detecting operation. Therefore, the gene detecting method is suitable in a medical or molecular biological screening test.

Examples of a test sample include humor such as blood, cerebrospinal fluid, tissue fluid, living body tissue taken by biopsy, living body secreta such as spewed sputum, nasal pharynx secreta, urethral and vaginal secreta, pus, living body excrement such as feces, urine, foods, drinks, soils, ground water, seawater, lake marsh water. Microorganisms and cells are usually contained in these test samples and, accompanying this, a polynucleotide such as DNA and RNA are present, or not present in some cases. Examples of a polynucleotide include genome, mitochondrial DNA, rRNA, tRNA, mRNA, viral RNA, plasmid, and fragments thereof of microorganisms and cells.

This gene detecting method is most useful in identifying (specifying) pathogens and microorganisms which has caused infectious disease or food poisoning. Examples of the pathogen include virus, rickettsia, chlamydia, mycoplasma, bacterium, fungus, spirochete, protozoan and reptiles.

When the gene detecting method is utilized in identifying a pathogen which has caused infectious disease or food poisoning, examples of a test sample include humor, living body tissue, living body secreta, living body excrement of an infected patient, or infection media such as foods, drinks, soils, ground water, seawater, lake marsh water that are thought to be an infection route. A target base sequence is a base sequence selected from a polynucleotide such as DNA and RNA accompanying with a plurality of pathogens which are predicted to have caused infectious disease or food poisoning. A preferable base sequence is inherent to each of a plurality of pathogens,

A base sequence inherent to each pathogen is a specific base sequence which does not hybridize with a polynucleotide such as genome, mitochondrial DNA, and RNA contained in other pathogens. Homology between a specific base sequence and a polynucleotide is preferably less than 50%, more preferably less than 40%, further preferably less than 30%. When homology between a base sequence inherent to each pathogen and a polynucleotide contained in other pathogens is 50% or more, it is impossible to specify species of the pathogens to only one kind based on the base sequence inherent to each pathogen. In addition, each pathogen and other pathogens are different in species, preferably different in subspecies.

As a base sequence inherent to each pathogen, a base sequence which is substantially identical to the base sequence may be used. A substantially identical base sequence is a base sequence which hybridizes with a base sequence inherent to each pathogen under the stringent conditions, and homology between both base sequences is preferably 80% or more, more preferably 90% or more, further preferably 95% or more, most preferably 98% or more. When homology between both base sequences is less than 80%, since those base sequences may not hybridize under the stringent conditions, the detection sensitivity is remarkably low.

This gene detecting method comprises a gene amplifying step of amplifying a polynucleotide comprising a target base sequence by the PCR reaction, and a gene detecting step of detecting whether a polynucleotide comprising a target base sequence is present in the amplified PCR reaction product. In the gene detecting step, for example, the presence of a polynucleotide is detected using a DNA microarray.

In the gene amplifying step, the PCR reaction is performed using a sample extract obtained by extraction from a test sample according to a polynucleotide extracting method, a gene detecting primer reagent for amplifying a polynucleotide comprising a target base sequence, and a labeling nucleotide. The sample extract is an extract obtained by extracting and purifying a polynucleotide contained in a test sample according to the known polynucleotide extracting method for extracting and purifying a DNA or an RNA, and is utilized as a template in the PCR reaction.

A gene detecting primer reagent is a mixture in which 10 or more kinds of amplifying primers, such as PCR primers, are mixed. Each amplifying primer is one pair of primers (having a chain length of, preferably, around 18 to 30 bases) which bind to a front end and a rear end of a polynucleotide comprising a target, base sequence, respectively. Each amplifying primer amplifies a polynucleotide comprising a target base sequence.

A gene detecting primer reagent is a mixture of preferably 10 to 400 kinds, more preferably 30 Lo 400 kinds, still more preferably 50 to 400 kinds, further preferably 50 to 100 kinds of amplifying primers. When the kind of amplifying primers in a gene detecting primer reagent is less than 10 kinds, since the kind of polynucleotides which can be detected by one time gene detecting operation is small, it is impossible to identify a pathogen which is predicted to be contained in a test sample. For this reason, it becomes necessary to perform a plurality of gene detecting operations, and not only the operation efficacy is extremely decreased, but also the cost for a test is remarkably increased.

According to the general microarray techniques, 96 to 384 kinds of spots are fixed on one glass slide. Then, the kind of amplifying primers suitable in a gene detecting method of the present embodiment is 80 to 96 kinds (more preferably 90 to 96 kinds), or 300 to 384 kinds (more preferably, 350 to 384 kinds). More economical is 80 to 96 kinds of amplifying primers.

It is preferred that a gene detecting primer reagent contains amplifying primers which are selected as follows. First, the currently known pathogens are classified into a plurality of groups depending on the symptom and the infection route. Each group contains all pathogens (10 kinds or more) causing the symptom inclusive. It is preferred that all amplifying primers for amplifying a polynucleotide inherent to each of pathogens belonging to each group are contained inclusive. In the following description, a gene detecting primer reagent made for identifying a pathogen is particularly referred to as pathogen identifying primer reagent.

As a labeling nucleotide, a labeling nucleotide applicable to a DNA microarray method can be used. A labeling nucleotide is most preferably a nucleotide (monomer) labeled with a fluorescent substance (Cy3, Cy5 etc.) or biotin because it has a high detecting sensitivity and is easily handled. A labeling nucleotide is taken into a polynucleotide to be amplified by the PCR reaction, and labels the amplified polynucleotide. In the case of a nucleotide labeled with biotin, a labeled polynucleotide is visualized by enzyme-linked immunosorbent assay (ELISA).

in a gene detecting step, the PCR reaction product obtained in the gene amplifying step is detected by a DNA microarray method. More particularly, as shown in Fig. 1, 10 or more kinds of target DNAs are fixed as different spots 22 on one substrate 21 (glass slide or plastic plate) to make a DNA microarray 20 (DNA chip). Using the DNA microarray 20, a polynucleotide contained in the FCR reaction product is tested. A target DNA is a DNA having a target base sequence or a DNA substantially identical to the DNA. Preferable detecting means in a gene detecting step is a laser fluorescents detecting apparatus or ELISA.

It is preferred that the DNA microarray 20 has spots 22 containing all target DNAs provided with a polynucleotide inherent to each pathogen belonging to a group inclusive, in which 10 or more kinds of pathogens which are known to be common or approximate in the symptom or the infection route of infectious disease or food poisoning are grouped as one group. In the following explanation, a DNA microarray made for idenbifying a pathogen is particularly called a pathogen identifying DNA microarray.

A substantially identical DNA is a DNA which hybridizes with a DNA comprising a target base sequence under stringent conditions, and homology between base sequences of both DNAs is preferably 80% or more, more preferably 90% or more, further preferably 95% or more. When homology between these both base sequences is less than 80%, since both base sequences may not hybridize under the stringent conditions, the detection sensitivity is remarkably decreased.

Then, a gene detecting method is explained.

For example, when infectious disease having unclear cause occurs, a gene detecting method is used for identifying a pathogen which has caused that infectious disease. First, a professional such as medical doctor predicts the cause for infectious disease (pathogen) from the symptom and the infection route. A test sample is taken from the infection route. Tf necessary, a pathogen in a test sample may be cultured and grown. A sample extract is extracted from a test sample.

As shown in Fig. 1, a test kit 100 (a primer reagent 10 and a DNA microarray 20) corresponding to the symptom is prepared. Alternatively, from a plurality of pathogen identifying primer reagents and a plurality of pathogen identifying DNA microarrays which have been made corresponding to the symptom and the infection route of a plurality of infectious diseases in advance, respectively, a predetermined pathogen identifying primer reagent and a pathogen identifying DNA microarray corresponding to the symptom and the infection route may be selected. For example, when the symptom is fever and cough, cold is suspicious, but a possibility of influenza and regionera pneumonia is also considered. Then, using a primer reagent and a DNA microarray for fever and cough, a pathogen is specified. The primer reagent contains a amplifying primer for amplifying a gene of a pathogen of a predetermined pathogen group containing influenza virus and regionera bacterium. The DNA microarray has a spot of a target DNA provided with a polynucleotide inherent to a pathogen of a predetermined pathogen group.

In order to perform a gene amplifying step, a sample extract, a pathogen identifying primer reagent, a labeled nucleotide and other reagents and enzymes necessary for the PCR reaction are PCR-reacted in one reaction tube. By the PCR reaction, a polynuclcotide is amplified.

when a predicted pathogen is not present in the sample extract, and when a polynucleotide is not present in the sample extract, the amplified polynucleotide is not present in the PCR reaction product, and a polynucleotide is not detected in the subsequent gene detecting step either.

On the other hand, when a predicted pathogen is present in the sample extract, a polynucleotide derived from that pathogen functions as a template in the PCR reaction. The PCR reaction proceeds by binding of the corresponding amplifying primer in a pathogen identifying primer reagent to the template, whereby a polynucleotide comprising a base sequence inherent to a pathogen is amplified. A labeling nucleotide is taken into the amplified polynucleotide (DNA), that is, the PCR reaction product. Therefore, the PCR reaction product is labeled.

Then, the labeled PCR reaction product is allowed to stand on a pathogen identifying DNA microarray. A target DNA fixed to a microarray and a polynucleotide (DNA) in the PCR reaction product are made to be hybridized. By visualizing a labeled polynucleotide hybridized on the pathogen identifying DNA microarray using the detection means such as ELISA, a pathogen contained in the test sample is identified.

According to the present embodiment, the following advantages are obtained.

The gene detecting method comprises a gene amplifying step of performing the PCR reaction using a sample extract obtained by extracting from a test sample according to a polynucleotide extracting method, and a gene detecting primer reagent for amplifying a target base sequence, and a gene detecting step of detecting the PCR reaction product obtained in the gene amplifying step with a DNA microarray. The gene detecting primer reagent contains 10 or more kinds of amplifying primers. The DNA microarray is such that a DNA having a base sequence to be amplified by each amplifying primer or a DNA substantially identical to the DNA is spotted thereon. In the gene amplifying step, 10 or more kinds of different base sequences are amplified at the same time.

In the gene detecting method, a polynucleotide in a test sample is effectively amplified utilizing the PCR reaction. The amplified polynucleotide is suitable for large scale screening, and is detected utilizing the DNA microarray techniques having high detection sensitivity. Therefore, whether a polynucleotide having a target base sequence is present in a test sample can be detected easily and at a high detection sensitivity. The presence of 10 or more kinds of polynucleotides having a target base sequence can be confirmed easily and rapidly. Since the detection result can be obtained in an extremely short time, the present gene detecting method is extremely useful in the medical field and the food hygiene field for which a pathogen must be examined as soon as possible.

Since 10 or more kinds of different base sequences can be tested by one time detection operation, a remarkably effective screening test becomes possible. Therefore, a pathogen which has caused infectious disease or food poisoning can be specified by approximately one time detection operation. Recently, the infectious disease is remarkably diversified in advanced countries, it has become difficult to diagnose it. Since a pathogen which has caused the infectious disease can be easily identified according to the present gene detecting method, the method is particularly useful in the medical field.

In order to perform similar large scale screening to the gene detecting method of the present embodiment, conventionally, it has been necessary to perform a plurality of times of detecting operations according to the Multiplex PCR method. Or, it has been necessary to perform the PCR reaction in a plurality of reaction tubes, and visualize the reactions by a plurality of electrophoresis gels. Therefore, large scale screening has been troublesome and required a long time. In addition, in the conventional Multiplex PCR method, there has been a possibility that an artificial mistake occurs in adding up the test results.

Conventionally, when food poisoning occurs and the cause for the food poisoning is predicted to be a bacterial pathogen, whether a predicted pathogen is present in an infection sample has been examined by culturing the sample on a predetermined nutrient medium. However, according to the conventional method, it has taken at least a few days until results are obtained. For that reason, prevention of infection expansion has been delayed in some cases. In addition, when it is difficult to predict a pathogen from the symptom or the infection route, there has been a defect that it is difficult to determine the culturing conditions such as selection of a medium and investigation becomes insufficient. To the contrary, according to the gene detecting method of the present embodiment, since a pathogen can be specified extremely rapidly, the method is also useful in the food hygiene field.

Since 10 to 100 kinds of amplifying primers are mixed in a gene detecting primer reagent, almost all kinds of pathogens which are predicted from the symptom or infection route can be screening-tested inclusive at sufficient detection sensitivity by one time detection operation.

Since the gene detecting method can be applied to a test sample such as humor, living body tissue, living body secreta, living body excrement, foods, drinks, soils, ground water, seawater and lake marsh water, a cause bacterium can be easily specified by testing various kinds of infection media which are considered to be the infection route for the infection disease and food poisoning. More particularly, except for a step of extracting a polynucleotide from a test sample according to a polynucleotide extracting method, operation procedures are constant. For this reason, since a worker easily learns test operation, he or she can effectively perform each step, and occurrence of mistake or confusion is prevented.

Since a gene detecting primer reagent contains a amplifying primer for amplifying a base sequence inherent to a pathogen which is predicted to have caused infection disease or poisoning, narrowing and identification of a pathogen is easy and certain. When a base sequence inherent to a pathogen encodes a gene essential for existence of the pathogen or a gene fragment thereof, a base sequence which is difficult to undergo a change such as mutation and is conserved, is detected. Therefore, omission of the detection result is reduced.

Pathogens are classified into a plurality of groups depending on the symptom and the infection route. A plurality of kinds of gene detecting primer reagents and DNA microarrays are prepared so as to correspond to a plurality of groups. A gene detecting primer reagent and a DNA microarray which are selected depending on the symptom, are used. Therefore, among pathogens which are predicted from the symptom and the infection route, true pathogens can be certainly and thoroughly identified.

Since the infection disease and food poisoning must be dealt with urgently, it is possible to deal with the urgent event rapidly by preparing a pathogen detecting primer reagent and a pathogen detecting DNA microarray in advance depending on a plurality kinds of symptoms and infections routes.

The present invention will be explained in more detail by way of the following Examples.

### (Example 1)

Each of 35 kinds of amplifying primers for amplifying a gene of a pathogen shown in Table 1 was prepared. Each of 12 kinds of amplifying primers for amplifying a gene of a pathogen shown in Table 2 was prepared. In addition, genes of 47 kinds of pathogens shown in Table 1 and Table 2 were spotted on a glass slide at 47 places to prepare a pathogen identifying DNA microarray.

**Table 1**

| Genes to be mixed for diagnosing respiratory infectious disease (Group A) | |
|---|---|
| Pathogens | Genes |
| Aspergillus group. | Group(185 rRNA) |
| Bacillus antracis CapA gene | Capsular gene:CapA |
| Bacillus antracis pag | Specific antigen protective antigen |
| Bacillus antracis Protective | Specific antigen (Protective antigen) |
| Bacillus antracis slime | Specific antigen (Protective antigen) |
| Bordetella pertussis 16SRNA | 16S rDNA |
| Bordetella pertussis toxin | Specific (Toxin) |
| Branhamella catarrhalis | 16S rRNA |
| Burkholderia cepacia group | Group(16SrRNA) |
| Candida spp. | 18S rDNA |
| Chlamydophila/Chlamydia spp. | Group (16S rRNA) |
| Corynebacterium diphteriae group | 16S rDNA |
| Corynebacterium diphteriae | Toxin |
| Coxiella burnetii | Specific(antigen) |
| Cryptococcus 5pp. | 18S rDNA |
| Fungus universal | Group (18S rRNA) |
| Haemophilus influenzae | Specific (16S rRNA) |
| Haemophilus influenzae antigen | Antigenic gene |
| Histoplasma capsulantum | Group (18S rRNA) |
| Legionella group | 16S rDNA |
| Legionella specific antigen | Specific antigen |
| Mycobacterium spp. | Group (16S rRNA) |
| Mycobacterium tuberculosis | Antigenic gene |
| Mycoplasma pneumoniae lipoprotein | Antigenic gene |
| Mycoplasma pneumoniae | Specific(16S rRNA) |
| Orientia tsutsugamushi 16SRNA | 16S rDNA |
| Orientia tsutsugamushi virulent | Specific(virulence) |
| Pneumocystis carinii | Specific antigen MSG 102 |
| Pseudomonas aeruginosa group | Group (16S rRNA) |
| Rickettsia/Ehrlchia spp. | Group(16S rRNA) |
| Staphylococcus aureus mecA | Specific(Virulence) |
| Staphylococcus aureus TSST-1 | Toxin |
| Streptococcus pneumoniae | Specific (virulence) |
| Streptococcus pyogenes virulence | Virulent gene |
| Yersinia pestis | Specific(virulence) |

**Table 2**

| Genes to be mixed for diagnosing respiratory infectious disease (Group B) | |
|---|---|
| Pathogens | Genes |
| Influenzae virus A | Specific(Virulence) |
| Influenzae virus B | Specific(antigen) |
| Influenzae virus C | Specific(antigen)RNA |
| RS virus 1 | Specific(antigen) |
| RS virus 2 | Antigenic gene |
| Herpes simplex virus 1 | Antigenic gene |
| Herpes simplex virus 2 | Antigenic gene |
| CM virus | Specific (Virulence) |
| Adenovirus 1 | Capsid gene |
| Adenovirus 2 | Capsid gene |
| Parainfluenza virus | Specific(antigen) |
| Measles virus | Specific(Virulence) |

47 kinds of amplifying primers for amplifying genes of 47 kinds of pathogens in Table 1 and Table 2, respectively, were classified into two groups of RNA virus and others, and two kinds of pathogen identifying primer reagents, that is, a first amplifying primer mixture of the RNA virus group, and a second amplifying primer mixture of others, were prepared. Every pathogen identifying primer reagent contains 10 or more kinds of amplifying primers.

A polynucleotide was extracted from spewed sputum taken from a patient who had been diagnosed to be respiratory infectious in the presence of an RNase inhibitor. A part of the polynucleotide was mixed with one of pathogen identifying primer reagents and Cy5 labeled base (nucleotide), and the PCR reactions was performed. Another part of the polynucleotide was mixed with another pathogen identifying primer reagent and Cy5 labeled base (nucleotide), and the PCR reaction was performed. First, one of the PCR reaction products was allowed to stand on a pathogen identifying DNA microarray, to form a hybrid with a target DNA. After washing, each spot on the DNA microarray was irradiated with laser using a laser fluorescence detecting apparatus, and the presence or absence of the amplified gene in which the Cy5 labeled base was incorporated, was examined. Subseguently, regarding another PCR reaction product, the presence or absence of the amplified gene was examined similarly.

As a result, formation of a hybrid with one kind of a target DNA on the pathogen identifying DNA microarray was clearly confirmed. The one kind of target DNA was consistent with a pathogen which is the cause for respiratory infectious disease diagnosed by a medical doctor that a patient was affected with. Therefore, by a method using the pathogen identifying primer reagent containing 10 or more kinds of amplifying primers, and the DNA microarray, a true pathogen was identified comparatively easily and rapidly among 47 kinds of pathogens.

### (Example 2)

Pathogens of group A shown in Table 1 cause a certain kind of respiratory infectious diseases having similar symptom and infection route. In Example 2, by mixing 35 kinds of amplifying primers for amplifying genes (shown in Table) inherent to each pathogen belonging to the group A, respectively, a pathogen identifying primer reagent was prepared. The pathogen identifying primer reagent of Example 2 is used for identifying a true pathogen when suspected of the respiratory infectious disease.

### (Example 3)

Pathogens of the group B shown in Table 2 cause respiratory infectious diseases exhibiting similar symptom to that of cold. By mixing 12 kinds of amplifying primers for amplifying genes (shown in Table) inherent to each pathogen belonging to the group B, respectively, a pathogen identifying primer reagent was prepared. The pathogen identifying primer reagent of Example 3 is used for identifying a true pathogen when suspected of respiratory infectious disease similar to cold.

### (Example 4)

Pathogens of the group C shown in Table 3 are RNA viruses which cause viral infectious disease. By mixing 67 kinds of amplifying primers for amplifying genes (shown in Table) inherent to each of RNA viruses belonging to the group C, respectively, a pathogen identifying primer reagent was prepared. The pathogen identifying primer reagent of Example 4 is used for identifying a true pathogen RNA virus when suspected of viral infectious disease.

**Table 3**

| Pathogens for which primers are mixed for viruses detection (Group C) | |
|---|---|
| RNA chikungunya virus | RNA Western equine encephalitis virus |
| RNA Dengue virus 1 | RNA Yellow fever virus |
| RNA Dengue virus 2 | RNA Batai virus |
| RNA Dengue virus 3 | RNA Bunyamwera virus |
| RNA Dengue virus 4 | RNA California encephalitis virus |
| RNA Encephalomyocarditis virus | RNA Crimean-Congo hemorrhagic |
| RNA Enterovirus4(68-71) | fever virus |
| RNA Foot-and mouth disease virus | RNA Ebora virus |
| RNA Gibbon ape leukemia virus | RNA Hantaan virus (Hemorrhagic fever with renal syndrome) |
| RNA Hepatitis A virus | RNA Human papillomavirus A (16, |
| RNA Hepatitis C virus | 18, 33, 58) |
| RNA Hepatitis E virus | RNA Human papillomavirus B (6, 11, 16, 18, 31, 33, 42, 52, 58) |
| RNA HIV-1:Human immunodeficiency virus 1 (AIDS virus) | RNA Human parainfluenza virus 1 |
| RNA HIV-2:Human immunodeficiency virus 2 | RNA Human respiratory syncytical virus (RS virus) |
| RNA Human coronavirus 229E | RNA Influenza A virus |
| RNA Human coxsachievirus A1 to 22 | RNA Influenza B virus |
| RNA Human coxsachievirus B1 to 6 | RNA Influenza C virus |
| RNA Human echovirus 1 to 7, 9, 11- | RNA Junin virus (Argentine |
| 27, 29-33, 68-71 | hemorrhagic fever) |
| RNA Human Rhino virus 1A | RNA Lassa virus |
| RNA Human T-lymphotropic virus 1 | RNA lymphocytic choriomeningitis virus |
| RNA Human T-lymphotropic virus 2 | RNA Machupo virus (Bolivian hemorrhagic fever) |
| RNA Japanese encephalitis virus | RNA Marburg virus |
| RNA Kunjin virus (Jpn encephalitis virus group) | RNA Measles virus |
| RNA Murray Valley encephalitis virus (Jpn encephalitis group) | RNA Mumps virus |
| RNA Negishi virus | RNA Parainfluenzavirus type 1 |
| RNA o'nyong-nyong virus | RNA rabies virus (RABV) |
| RNA Polio virus 1 | RNA Rift Valley fever virus |
| RNA Powassan virus | RNA vesicular stomatitis virus |
| RNARhinovirus | RNA bluetongue virus 1 |
| RNA Rubella virus | RNA Colorado tick fever virus (CTFV) |
| RNA St.Louis encephalitis virus | RNA HHV2: Human herpex simplex virus2 |
| RNA Tick-borne encephalitis virus | RNA Human rocavirus 1 |
| RNA Venezuelan equine encephalitis virus | RNA parvovirus B19 |
| RNA West Nile Fever virus (Jpn | RNA Hepatitis D virus |
| encephalitis group) | RNA Hepatitis G virus |

### (Example 5)

Pathogens of the group D shown in Table 4 are DNA viruses which cause viral infectious disease. By mixing 16 kinds of amplifying primers for amplifying genes (shown in Table) inherent to DNA viruses belonging to the group D, respectively, a pathogen identifying primer reagent was prepared. The pathogen identifying primer reagent of Table 5 is used for identifying a true pathogen DNA virus when suspected of viral infectious disease.

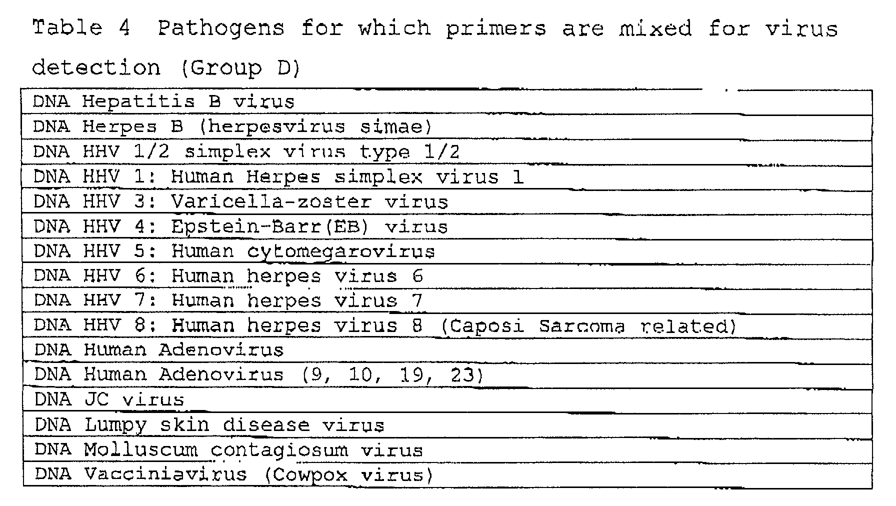

### (Example 6)

By spotting genes inherent to each of 83 kinds of pathogens shown in Table 3 and Table 4 on one glass slide, a pathogen identifying DNA microarray was prepared. The DNA microarray of Example 6 is used for identifying a true pathogen virus when suspected of viral infectious disease.

### (Example 7)

Pathogens of the Group E shown in Table 5 are genes of Salmonella bacteria which cause food poisoning. By mixing 15 kinds of amplifying primers for amplifying genes related to each of O antigen and H antigen of Salmonella bacteria belonging to the group E, respectively, a gene detecting primer reagent was prepared. The pathogen identifying primer reagent of Example 7 is used for classifying serological bacterium type of Salmonella bacteria when suspected of food poisoning.

**Table 5**

| Genes for identifying and detecting Salmonella (Group E) | |
|---|---|
| O antigen | rfb E(09) |
| | rfb J(02) |
| | rfb S(02) |
| Capsule | vipR(inner) |
| | vipR(outer) |
| Flagellar antigen | FliC;a |
| | FliC:b |
| | FliC:c |
| | FliC:d |
| | FliC:i |
| | FliC:gmp |
| | FLAGELLIN 1 |
| | FLAGELLIN 2 |
| disease factor | inv A |
| | Enterotoxin |

It should be apparent to those skilled in the art that the present invention may be embodied in many other specific forms without departing from the spirit or scope of the invention. For example, the present embodiment may be modified as follows.

A amplifying primer for amplifying a polynucleotide comprising another inherent base sequence contained in species (pathogen) derived from a polynucleotide which is amplified by each amplifying primer may be additively mixed into a pathogen identifying primer reagent. In this case, it becomes possible to identify species of a pathogen by one time detection operation, and further confirm it.

A DNA microarray having spots of pathogens of a plurality of groups may be prepared. According to the DNA microarray, a plurality of kinds (groups) of infectious diseases can be tested at once.

In a gene amplifying step, a labeled nucleotide and an unlabeled nucleotide may be mixed to perform the PCR reaction.

A gene detecting method may be utilized for identifying contaminating bacteria (microorganisms) which has caused soil contamination and water contamination (red tide etc.) occurring in soils, ground water, seawater, and lake marsh water.

When a test sample is human living body tissue taken by biopsy, a gene used in gene diagnosis for testing an oncogene or single nucleotide polymorphism (SNP) or a gene fragment thereof may be spotted on a DNA microarray. In this case, the findings regarding mutation of a gene which is the cause for cancer can be obtained easily and rapidly, and this can serve for therapy. In addition, since the findings regarding SNP can be examined easily and rapidly, it can serve upon drug administration and treatment in view of individual difference.

A gene detecting primer reagent may be prepared in advance, for which test samples (infection media) such as humor (blood, cerebrospinal fluid, tissue fluid), living body tissue, living body secreta (spewed sputum, nasal pharynx secreta, urethral and vaginal secreta, pus), living body excrement (feces, urine), foods, drinks, soils, ground water, seawater, lake marsh water are classified.

Alternatively, a gene detecting primer reagent may be prepared in advance, which is made to correspond to the symptom, the infection route, the development area, race, a gene or a combination thereof.

Each amplifying primer may amplify an RNA. The present examples and embodiments are to be considered as illustrative and not restrictive and the invention is not to be limited to the details given herein, but may be modified within the scope and equivalence of the appended claims.

## Claims

1. A method for determining whether a polynucleotide having a target base sequence is present in a test sample, **characterized by**:
preparing a gene detecting primer reagent by mixing at least ten kinds of amplifying primers;
preparing a DNA microarray by spotting DNAs having a base sequence to be amplified by the amplifying primers, respectively, or DNAs substantially identical to the DNAs on a substrate;
extracting a sample extract from the test sample;
amplifying a gene by performing the PCR reaction using the sample extract and the gene detecting primer reagent; and
detecting the PCR reaction product using the DNA microarray.

2. The method according to claim 1, wherein the step of preparing the gene detecting primer reagent includes a step of mixing 10 to 100 kinds of amplifying primers,

3. The method according to claim 1, wherein the test sample is selected from a group consisting of humor, living body tissue, living body secreta, living body excrement, food, drink, soil, ground water, seawater and lake marsh water, and wherein the step of preparing the gene detecting primer reagent includes a step of selecting a amplifying primer for amplifying a base sequence inherent to a pathogen of infectious disease or food poisoning, as one of the at least ten kinds of amplifying primers.

4. The method according to claim 3, wherein the step of preparing the gene detecting primer reagent includes a step of selecting a amplifying primer for amplifying another base sequence inherent to the pathogen, as one of the at least ten kinds of amplifying primers.

5. The method according to claim 3, wherein the step of preparing the gene detecting primer reagent includes a step of selecting a amplifying primer for amplifying a base sequence inherent to each of a plurality of pathogens belonging to a predetermined pathogen group corresponding to a predetermined test purpose, among a plurality of pathogen groups corresponding to a plurality of test purposes, respectively, as one of the at least ten kinds of amplifying primers, and wherein the pathogens are classified into a plurality of groups depending on the symptom and the infection route.

6. The method according to claim 1, wherein the test sample is human living body tissue, and the DNA is a gene for gene diagnosis or a fragment thereof.

7. The method according to claim 6, wherein the gene used for gene diagnosis is one of an oncogene and a gene for single base polymorphism diagnosis.

8. The method according to claim 1, wherein the target base sequence is one of a base sequence encoding a gene essential for the existence of a pathogen and a gene fragment thereof.

9. The method according to claim 1, wherein the gene amplifying step includes performing the PCR reaction by adding a labeling nucleotide.

10. A gene detecting primer reagent (10) for detecting a polynucleotide having a target base sequence in a test sample, the primer reagent is **characterized by** a mixture of at least ten kinds of amplifyinq primers.

11. A DNA microarray (20) for detecting a polynucleotide having a target base sequence in a test sample, **characterized by**:
spots (22) of DNAs having a base sequence to be amplified by each of ten or more kinds of amplifying primers, or of DNAs substantially identical to the DNAs, formed on a substrate (21).

12. A test kit (100) for detecting a polynucleotide having a target base sequence in a test sample, **characterized by**:
a primer reagent (10) obtained by mixing at least ten kinds of amplifying primers; and
a DNA microarray (20) having spots (22) of DNAs having a base sequence to be amplified by each of the amplifying primers, or of DNAS substantially identical to the DNAs.

13. A method of manufacturing a test kit for detecting a polynucleotide having a target base sequence in a test sample, **characterized by** the steps of:
preparing a gene detecting primer reagent by mixing at least ten kinds of amplifying primers; and
preparing a DNA microarray by spotting DNAs having a base sequence to be amplified by the amplifying primers, or DNAs substantially identical to the DNAs on a substrate.

14. , A test kit for specifying a pathogen of infectious disease, **characterized by**:
a primer reagent obtained by mixing a plurality of amplifying primers for amplifying a base sequence inherent to a plurality of pathogens causing similar symptom to symptom of the infectious disease, respectively; and
a DNA microarray having DNA spots having a polynucleotide inherent to the plurality of pathogens,

15. The test kit according to claim 14, wherein the plurality of amplifying primers comprise at least ten primer pairs.

16. The test kit according to claim 15, wherein the plurality of DNA spots comprise spots of at least ten kinds of target DNAs.
